# EUROPEAN PATENT SPECIFICATION

(11) **EP 1 778 399 B1**
(45) Date of publication and mention of the grant of the patent: **23.11.2011**
(21) Application number: 05761209.5
(22) Date of filing: 07.07.2005
(51) Int. Cl.: B01J 31/04, B01J 31/14, B01J 31/22, B01J 31/26, C07C 2/30, C08F 10/02, C08F 4/16, C08F 4/642

(54) **IMPROVED COCATALYST FOR THE PRODUCTION OF LINEAR ALPHA-OLEFINS**
VERBESSERTER COKATALYSATOR FÜR DIE HERSTELLUNG VON LINEAREN ALPHA-OLEFINEN
COCATALYSEUR AMELIORÉ DESTINÉ À LA PRODUCTION D'ALPHA OLÉFINES LINÉAIRES

(30) Priority: 20.08.2004 DE 102004040497
(43) Date of publication of application: 02.05.2007
(73) Proprietor: Linde AG, 80331 München (DE); Saudi Basic Industries Corporation, Riyadh 11422 (SA)
(72) Inventor: BÖLT, Heinz, 82515 Wolfratshausen (DE); FRITZ, Peter, Matthias, 82008 Unterhaching (DE); HACKNER, Holger, 80807 München (DE); ABURAQABAH, Atieh, Saudi Basic Industries Corp., 11551 Riyadh (SA); ZAHOOR, Mohammed, Saudi Basic Industries Corp., 11422 Riyadh (SA); MOSA, Fuad, Saudi Basic Industries Corp., 11551 Riyadh (SA)
(74) Representative: Winkler, Andreas Fritz Ernst
(86) International application number: PCT/EP2005/007321
(87) International publication number: WO 2006/018071

(56) References cited:
- DE-A1- 19 812 066
- FR-A- 2 689 500
- US-A- 3 862 257
- US-A- 4 434 312
- US-A- 4 966 874
- US-A- 5 449 850
- US-A1- 2002 147 375

## Description

The invention relates to a homogeneous catalyst for the production of linear alpha-olefins by oligomerisation of ethylene, consisting of a zirconium salt of organic acids and a cocatalyst.

Linear alpha-olefins (LAO), for example those with four to 30 carbon atoms, are compounds which are, for example, widely used and required in large quantities as comonomers for modifying the properties of polyolefins or as a starting material for the production of plasticisers, household cleansers, flotation agents, emulsifiers, drilling fluids, surface-active substances and synthetic oils, putties, and sealants.

According to the inventions described in USSR inventor's certificate 1042701 A, the unaccepted Italian patent application ITA24498A/79 and German patents DE4338414 and DE4338416, the oligomerisation of ethylene to C4-C30 LAO is performed in an organic solvent at 60 to 80°C and pressures of 2.0 to 4.0 MPa. Toluene, benzene or heptane are used as reaction media. All these processes make use of catalysts which consist of a zirconium salt of an organic acid and one of the alkylaluminiums Al(C₂H₅)₃, Al₂Cl₃(C₂H₅)₃ or AlC(C₂H₅)₂ as cocatalyst. In order to prevent the occurrence of unwanted secondary reactions, it is necessary to terminate the oligomerisation immediately the reaction mixture leaves the reactor. The catalyst is deactivated by addition of a polar oxygen compound (H₂O, methanol, ethanol, carboxylic acids etc.) to the reaction mixture and is then separated from the mixture by adsorption onto a regeneratable aluminium oxide gel. The deactivated catalyst must be replaced by fresh material.

The high price of the zirconium salt means that the catalyst accounts for a considerable proportion of the operating costs of the processes. Higher activity of the catalyst (expressed in kg of LAO product/g of Zr) results in lower consumption and thus in increased economic viability of LAO production because operating costs and, due to the consequently possible reduction in size of the apparatuses for storing, apportioning, deactivating and removing the catalyst, also capital costs are reduced.

US 2002/147375A1 discloses a process for the preparation of low molecular weight linear alpha-olefins having 4 to 24 carbon atoms, comprising oligomerizing ethylene in an inert aliphatic or aromatic solvent in the presence of a catalyst. The catalyst comprises a zirconium compound and a co-catalyst of alkyl aluminum and/or alkyl aluminum halide.

US 4,966,874 discloses linear alpha-olefins which are prepared by the oligomerization of ethylene using a two component catalyst system. One component thereof is an alkyl metal selected, e.g., from R₂AlX, wherein R is C1 - C20 alkyl and X is Cl or Br.

FR 2,689,500 discloses a process for oligomerization of ethylene utilizing a catalyst of a zirconium compound and an aluminum based co-catalyst of the formula RₙX₃₋ₙ.

US 4,434,312 discloses a catalyst for the oligomerization of ethylene comprising a dialkyl aluminum halide and a monoalkyl aluminum dihalide.

US 5,449,850 discloses the preparation of olefins by oligomerization of ethylene using a catalyst system comprising an alkyl metal selected from the group of R₂AlX, RAlX₂, R₃Al₂X₃ and R₃Al with R being C1 - C20 alkyl and X being Cl or Br.

It is accordingly an object of the present invention to increase the activity of the catalyst hitherto used for LAO production by modifying the chemical composition thereof in such a manner that lower catalyst consumption is achieved without any drop in product quality.

This object is achieved by the catalyst of claim 1. Preferred embodiments are disclosed in the subclaims.

DE4338414 describes a catalyst system which is conveniently used in connection with the process explained therein. The zirconium compound has the chemical formula ZrClₘXₙ, wherein m + n = 4.0 ≤ m ≤ 2 and X denotes a carboxylate derived from a C₄ to C₉ fatty acid. The compound Al₂Cl₃(C₂H₅)₃ has proved to be a particularly advantageous cocatalyst in practice, giving rise to a catalyst activity of 10 kg of (LAO) per 1 g of (Zr).

It has been found that the decisive parameter determining the level of catalyst activity is the molar ratio of chlorine to aluminium in the cocatalyst. Given suitable selection of this ratio, values considerably higher than 10 kg of (LAO)/g of (Zr) may be achieved. Product purity is simultaneously improved.

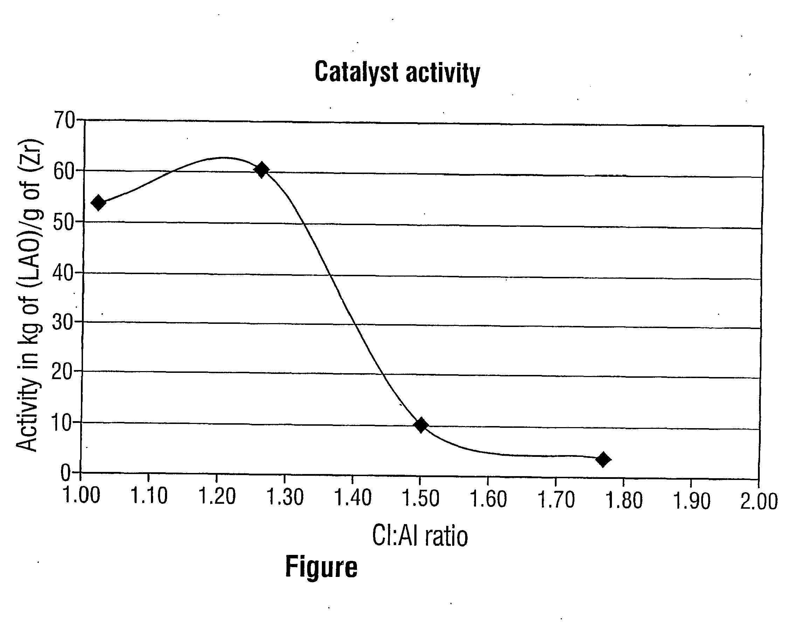

In the Figure, catalyst activity is plotted as a function of the Cl:Al ratio in the cocatalyst. Starting from an activity of 10 kg of (LAO)/g of (Zr) at a Cl:Al ratio of 1.5, corresponding to the prior art (Al₂Cl₃(C₂H₅)₃), activity rapidly rises as the value of the ratio drops until, at Cl:Al = 1.26, it is approx. 500% above the prior art. Activity then slowly drops back down to reach a value of approx. 54 kg of (LAO)/g of (Zr) at Cl:Al = 1.02. If Cl:Al ratios of higher than 1.5 are used, activity drops. At Cl:Al = 1.77, it is only 3.1 kg of (LAO)/g of (Zr).

## Claims

1. A homogeneous catalyst for the production of linear alpha-olefins by oligomerization of ethylene, consisting of a zirconium salt of organic acids and a cocatalyst, **characterized in that** the cocatalyst is produced as a mixture of alkylaluminium(s) and aluminium chloride (AlCl₃), wherein the molar ratio of chlorine to aluminium in the cocatalyst is adjusted to a value of between 1.0 and 1.5.

2. A catalyst according to claim 1, **characterized in that** the cocatalyst is produced as a mixture of the alkylaluminium(s) Al(C₂H₅)₃ and/or AlCl(C₂H₅)₂ and/or Al₂Cl₃(C₂H₅)₃ and/or AlCl₂(C₂H₅) and aluminium chloride (AlCl₃).

3. A catalyst according to either one of claims 1 or 2, **characterized in that** the ratio of chlorine to aluminium in the cocatalyst is adjusted to a value of between 1.0 and 1.3.

## Patentansprüche

1. Homogener Katalysator für die Herstellung von linearen alpha-Olefinen durch Oligomerisation von Ethylen, der aus einem Zirkoniumsalz organischer Säuren und einem Cokatalysator besteht, **dadurch gekennzeichnet, dass** der Cokatalysator hergestellt ist als eine Mischung aus Aluminiumalkyl(en) und Aluminiumchlorid (AlCl₃), wobei das Molverhältnis von Chlor zu Aluminium im Cokatalysator auf einen Wert zwischen 1,0 und 1,5 eingestellt ist.

2. Katalysator nach Anspruch 1, **dadurch gekennzeichnet, dass** der Cokatalysator hergestellt ist als eine Mischung der Aluminiumalkyl(e) Al(C₂H₅)₃ und/oder AlCl(C₂H₅)₂ und/oder Al₂Cl₃(C₂H₅)₃ und/oder AlCl₂(C₂H₅) und Aluminiumchlorid (AlCl₃).

3. Katalysator nach Anspruch 1 oder 2, **dadurch gekennzeichnet, dass** das Verhältnis von Chlor zu Aluminium im Cokatalysator auf einen Wert zwischen 1,0 und 1,3 eingestellt ist.

## Revendications

1. Catalyseur homogène pour la production d'alpha-oléfines linéaires par oligomérisation d'éthylène, composé d'un sel de zirconium d'acides organiques et d'un cocatalyseur, **caractérisé en ce que** le cocatalyseur est produit comme un mélange d'alkylaluminium(s) et de chlorure d'aluminium (AlCl₃), où le rapport molaire de chlore par l'aluminium dans le cocatalyseur est réglé à une valeur comprise entre 1,0 et 1,5.

2. Catalyseur selon la revendication 1, **caractérisé en ce que** le cocatalyseur est produit comme un mélange d'alkylaluminium(s) Al(C₂H₅)₃ et/ou de AlCl(C₂H₅)₂ et/ou de Al₂Cl₃(C₂H₅)₃ et/ou de AlCl₂(C₂H₅) et de chlorure d'aluminium (AlCl₃).

3. Catalyseur selon l'une des revendications 1 ou 2, **caractérisé en ce que** le rapport du chlore par l'aluminium dans le cocatalyseur est réglé à une valeur comprise entre 1,0 et 1,3.
